# EUROPEAN PATENT SPECIFICATION

(11) **EP 2 695 590 B1**
(45) Date of publication and mention of the grant of the patent: **06.12.2017**
(21) Application number: 12767373.9
(22) Date of filing: 21.03.2012
(51) Int. Cl.: A61F 13/15, A61F 13/49, A61F 13/494, A61F 13/53, A61F 13/496

(54) **DISPOSABLE ARTICLE OF CLOTHING**
EINWEG-BEKLEIDUNGSARTIKEL
VÊTEMENT JETABLE

(30) Priority: 01.04.2011 JP 2011082309
(43) Date of publication of application: 12.02.2014
(73) Proprietor: Unicharm Corporation, Ehime 799-0111 (JP)
(72) Inventor: ICHIKAWA, Makoto, Kanonji-shi Kagawa 769-1602 (JP); SASAYAMA, Kenichi, Kanonji-shi Kagawa 769-1602 (JP); KATSURAGAWA, Kunihiko, Kanonji-shi Kagawa 769-1602 (JP)
(74) Representative: Peter, Julian
(86) International application number: PCT/JP2012/057092
(87) International publication number: WO 2012/137598

(56) References cited:
- EP-A2- 0 648 482
- JP-A- 4 242 645
- JP-A- 10 243 961
- JP-A- 11 188 062
- JP-A- 2000 232 985
- JP-A- 2003 275 236
- JP-A- 2004 329 238
- JP-A- 2006 149 571
- US-A1- 2005 171 499
- US-A1- 2008 234 649

## Description

### {Technical Field}

The present invention relates to disposable wearing articles and more specifically to the disposable wearing articles such as disposable diapers, disposable toilet training pants, disposable incontinent pants and disposable sanitary pants adapted to prevent body exudates from leaking sideways beyond leg-opening peripheries and preventing the wearer's body from being soiled with the body exudates.

### {Background}

Conventionally, disposable wearing articles having an arrangement functioning to prevent the body exudates from leaking sideways beyond theleg-opening peripheries are known. For example, PTL 1 (JP 2005-87621 A) discloses a disposable wearing article including a chassis to which a liquid-absorbent structure is attached and leg elastic elements attached to the leg opening peripheries.

### {Citation List}

### {Patent Literature}

{PTL 1} : JP 2005-87621 A
Further prior art in this technical field is disclosed in documents US 2008/234649 A1, US 2005/171499 A1 and EP 0 648 482 A2.

### {Summary}

### {Technical Problem}

According to the wearing article disclosed in PTL 1, the leg elastic elements are composed of front leg elastic elements extending from a front side of the leg-opening peripheries to a central portion of a crotch region and rear leg elastic elements extending from a rear side of the leg-opening peripheries to the central portion of the crotch region so that the front and rear leg elastic elements intersect with each other in the central portion of the crotch region. With such an arrangement, the entire crotch region comes in close contact with the wearer's body and thereby prevents a displacement of the article during use thereof and prevents the body exudates from leaking beyond the leg-opening peripheries.

However, in this wearing article, the entire crotch region remains in close contact with the wearer's body even after occurrence of excretion and, inconsequence, the body exudates remains in contact with or adhering to the wearer's body for a relatively long time under unsanitary condition. Particularly when such wearing articles are used for infants and/or aged persons having sensitive skin, the wearers might suffer from skin troubles such as rash and eruption. In addition, during occurrence of excretion, the crotch region remains in close contact with the wearer's body and it is difficult for the article to retain a relatively large amount of body exudates. Consequently, there is a possibility that surplus body exudates might move to the back side of the wearer and soil the wearer's skin in a relatively large area.

An object of the present invention is to provide a disposable wearing article adapted to prevent body exudates from leaking sideways beyond a leg-opening periphery and to prevent the wearer's body from being soiled with the body exudates.

### {Solution to Problem}

The present invention provides a disposable wearing article according to claim 1.

According to an embodiment of the present invention, the liquid-absorbent structure is formed of superabsorbent polymer particles wrapped with a liquid-permeable sheet in a sheet configuration and a thickness dimension of the crotch region in the substantially central portion thereof including the liquid-absorbent structure is about 5.0 mm or less.

### {Advantageous Effects of Invention}

With the disposable wearing article according to one or more embodiments of the present invention, the lateral elastic regions of the crotch panel prevent body exudates from leaking sideways beyond the leg-opening peripheries. The liquid-absorbent structure has a relatively low stiffness and the crotch panel contains therein the containing space swollen shape adapted to contain a relatively large amount of body exudates. With such arrangement, upon occurrence of excretion, the crotch panel is spaced downwardly from the wearer's body under its own mass of body exudates and the wearer's body is unlikely to be soiled by body exudates.

### {Brief Description of Drawings}

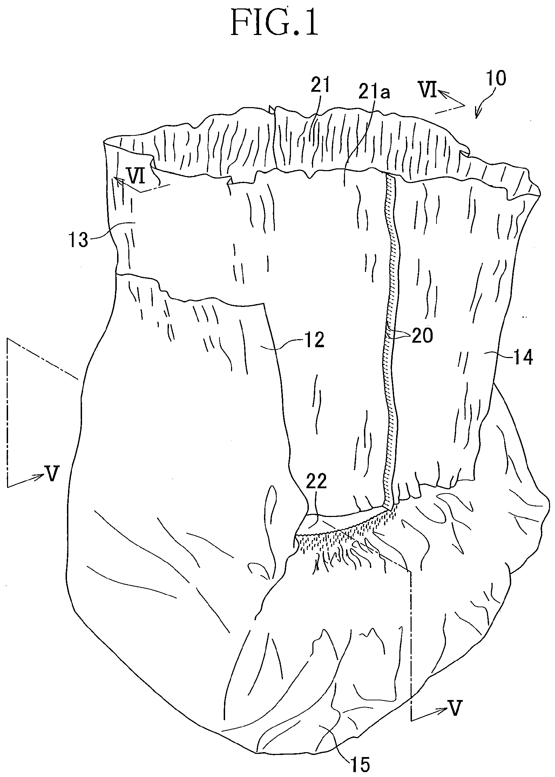

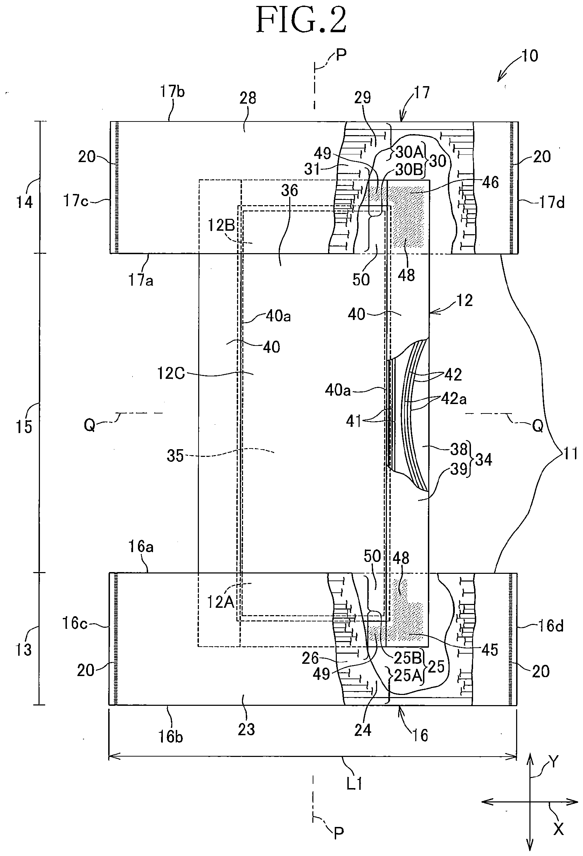

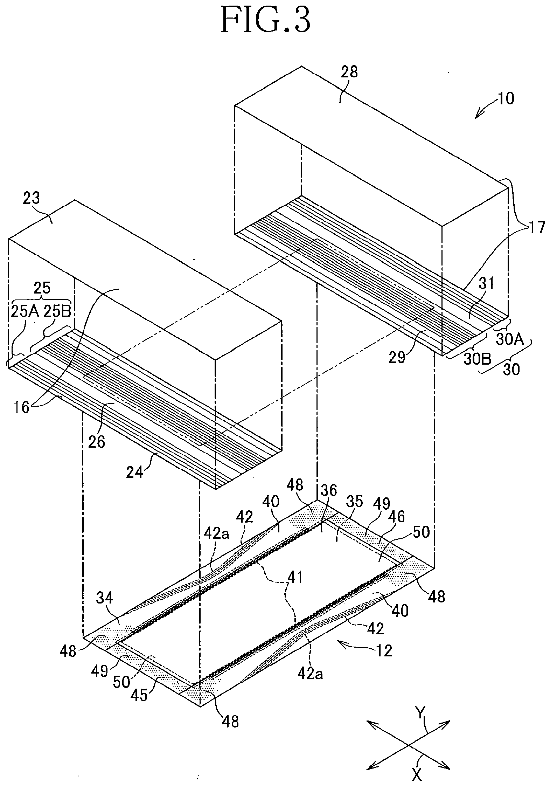

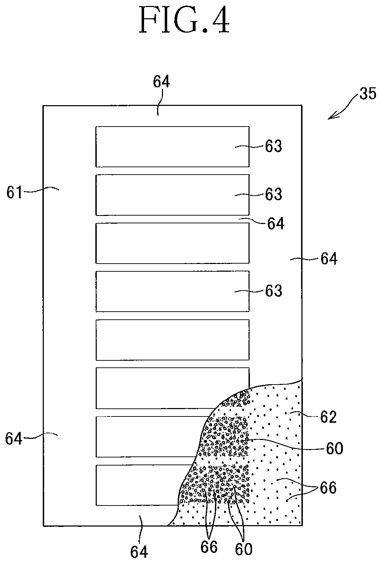

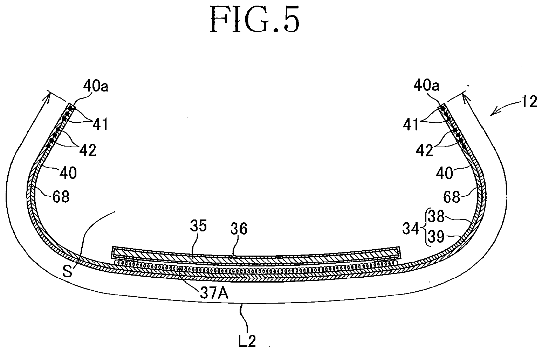

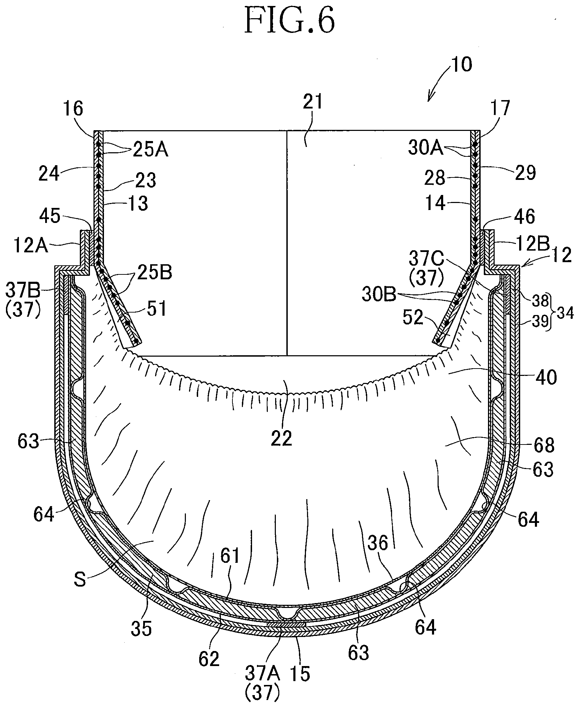

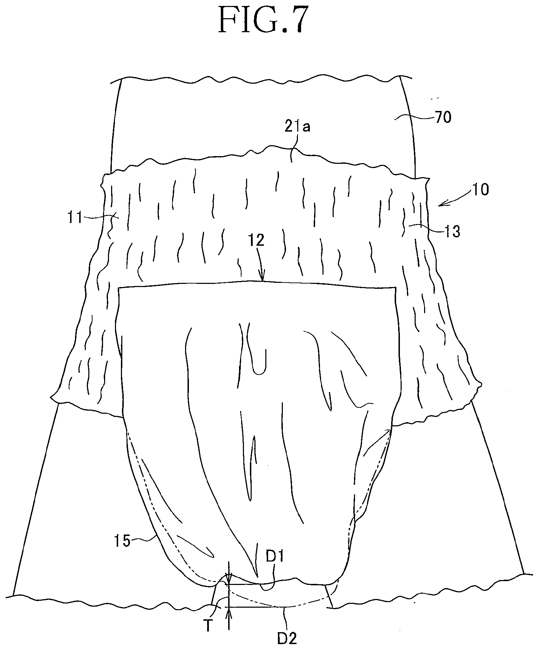

{Fig. 1} Fig. 1 is a perspective view of a disposable diaper as an example of a disposable wearing article according to the present invention.
{Fig. 2} Fig. 2 is a partially cutaway developed plan view illustrating the diaper developed in a front-back direction after front and rear waist regions have been peeled off from each other along side seams and viewed from an interior surface thereof.
{Fig. 3} Fig. 3 is an exploded perspective view of the diaper.
{Fig. 4} Fig. 4 is a partially cutaway perspective view of a liquid-absorbent structure.
{Fig. 5} Fig. 5 is a schematic sectional view taken along line V-V in Fig. 1.
{Fig. 6} Fig. 6 is a schematic sectional view taken along line VI-VI in Fig. 1.
{Fig. 7} Fig. 7 is a diagram illustrating an aspect of measurement to evaluate a sagging effect of a crotch panel.

### {Description of Embodiments}

Referring to Figs. 1 through 3, a disposable diaper 10 illustrated as an example of a disposable wearing article according to the present invention having a longitudinal direction Y, a transverse direction X being orthogonal to the longitudinal direction Y, an imaginary longitudinal center line P-P bisecting a width dimension in the transverse direction X and an imaginary transverse center line Q-Q bisecting a length dimension in the longitudinal direction Y.

The diaper 10 includes a skin-facing surface, a non-skin-facing surface opposed to the skin-facing surface, an annular elastic waist panel 11 circumferentially extending about the waist of the wearer, a crotch panel 12 attached to the non-skin-facing surface of the elastic waist panel 11, a front waist region 13, a rear waist region 14 and a crotch region 15 extending in the longitudinal direction Y between the front and rear waist regions 13, 14.

The diaper 10 is formed symmetrically about the imaginary center line P-P and the elastic waist panel 11 is formed of a front waist panel 16 defining the front waist region 13 and a rear waist panel 17 defining the rear waist region 14.

The front waist panel 16 has a transversely long rectangular shape contoured by an inner end edge 16b extending in the transverse direction X and intersecting with the crotch panel 12, an outer end edge 16b extending in the transverse direction X to be spaced apart from and opposed to the inner end edge 16a in the longitudinal direction Y and lateral edges 16c, 16d extending in the longitudinal direction Y between the inner and outer end edges 16a, 16b.

The rear waist panel 17 is substantially the same as the front waist panel 16 in shape as well as in size and has a transversely long rectangular configuration contoured by an inner end edge 17a extending in the transverse direction X and intersecting with the crotch panel 12, an outer end edge 17b extending in the transverse direction X to be spaced from and opposed to the inner end edge 17a and lateral edges 17c, 17d extending in the longitudinal direction Y between the inner and outer end edges 17a, 17b.

The lateral edges 16c, 16d of the front waist panel 16 are overlapped with the lateral edges 17c, 17d of the rear waist panel 17, respectively, and joined to each other by means of respective series of side seams 20 arranged at intervals in the longitudinal direction Y and thereupon a waist-opening 21 and a pair of leg-openings 22 are defined (See Fig. 1). The side seams 20 may be formed with use of the joint techniques of known art, for example, various types of heat sealing techniques such as thermal embossing/debossing and ultrasonic sealing techniques.

The front waist panel 16 includes a first interior sheet 23 lying on the side of the skin-facing surface and a first exterior sheet 24 lying on the side of the non-skin-facing surface. Each of the first interior and exterior sheets 23, 24 may be formed with use of a liquid-impermeable SMS (spunbonded/meltblown/spunbonded) fibrous nonwoven fabric, a spunbonded nonwoven fabric, a plastic sheet or a laminated sheet composed thereof, each having a mass per unit area of about 10 to 30 g/m². The both sheets 23, 24 are bonded to each other with a hot melt adhesive distributed at intervals to the interior surface of at least one of these two sheets or by the above-mentioned heat sealing technique.

Between the two sheets 23, 24, a plurality of strand or string front waist elastic elements 25 are arranged to extend in the transverse direction X. These front waist elastic elements 25 arranged in this manner make the front waist panel 16 elastically stretchable and contractible at least in the transverse direction X. In this regard, it is also possible to bond the sheets 23, 24 to each other only with a hot melt adhesive distributed to substantially the entire peripheral surfaces of the respective front waist elastic elements 25.

The front waist elastic elements 25 are composed of front-upper waist elastic elements 25A extending in the transverse direction X along the outer end edge 16b of the front waist panel 16 and front-lower waist elastic elements 25B extending in the transverse direction X along the inner end edge 16a of the front waist panel 16. In an upper part of this elasticized region, the front-lower waist elastic elements 25B are arranged more closely than the front-upper waist elastic elements 25A and an inelastic region 26 including none of the elastic elements is defined between the front-upper waist elastic elements 25A and the front-lower waist elastic elements 25B.

The rear waist panel 17 includes a second interior sheet 28 lying on the side of the skin-facing surface and a second exterior sheet 29 lying on the side of the non-skin-facing surface. Each of the second interior and exterior sheets 28, 29 may be formed with use of a liquid-impermeable SMS (spunbonded/meltblown/spunbonded) fibrous nonwoven fabric, a spunbonded nonwoven fabric, a plastic sheet or a laminated sheet composed thereof, each having a mass per unit area of about 10 to about 30 g/m². The second interior and exterior sheets 28, 29 are bonded to each other with a hot melt adhesive distributed to the interior surface of at least one of these two sheets or by means of the above-mentioned heat sealing technique.

Between the two sheets 28, 29, a plurality of strand or string rear waist elastic elements 30 are arranged to extend in the transverse direction X. These rear waist elastic elements 30 arranged in this manner make the rear waist panel 17 elastically stretchable and contractible at least in the transverse direction X. In this regard, it is also possible to bond the sheets 28, 29 to each other only with a hot melt adhesive distributed to substantially the entire peripheral surfaces of the respective rear waist elastic elements 30.

The rear waist elastic elements 30 are composed of rear-upper waist elastic elements 30A extending in the transverse direction X along the outer end edge 17b of the rear waist panel 17 and rear-lower waist elastic elements 30B extending in the transverse direction X along the inner end edge 17a of the rear waist panel 17. In an upper part of this elasticized region, the rear-lower waist elastic elements 30B are arranged more closely than the rear-upper waist elastic elements 30A and an inelastic region 31 including none of the elastic elements is defined between the rear-upper waist elastic elements 30A and the rear-lower waist elastic elements 30B.

Referring to Figs. 1 and 2, a length dimension L1 of the waist-opening peripheral edge 21a of the front waist region 13 (the rear waist region 14) measured with the front and rear waist regions 13, 14 being stretched in the transverse direction X is in a range of about 450 to about 550 mm for the M-size diaper 10 and in a range of about 550 to about 650 mm for the L-size diaper 10.

The crotch panel 12 has a longitudinally long substantially rectangular configuration contoured by a front end portion 12A attached to the non-skin-facing surface (i.e., exterior surface), a rear end portion 12B attached to the non-skin-facing surface of the rear waist panel 17 and an intermediate portion 12C extending in the longitudinal direction Y between the front and rear end portions 12A, 12B. The crotch panel includes a crotch laminated sheet 34, a liquid-absorbent structure 35 placed on the skin-facing surface (i.e., interior surface) of the crotch laminated sheet 34 and a bodyside liner 36 formed of a liquid-permeable sheet lying on the upper surface of the liquid-absorbent structure 35.

So long as advantageous effects of the present invention as will be described later can be assured, it is not essential to form the diaper 10 according to the present invention from the front and rear waist regions 13, 14 and the crotch region 15 which are separately prepared but it is also possible to form the diaper 10 from an integrated chassis. In this case, the liquid-absorbent structure 35 may be attached to the interior surface of the chassis or suspended from the interior surface of the chassis.

Referring to Fig. 6, the crotch laminated sheet 34 and the liquid-absorbent structure 35 are bonded to each other by means of a structure bonding region 37 formed of a hot melt adhesive distributed to opposed surfaces thereof. Specifically, the liquid-absorbent structure 35 is attached to the interior surface of the crotch laminated sheet 34 in a bonding region 37A located in a midsection of the crotch region 15 and in bonding regions 37B, 37C located in the front and rear waist regions 13, 14 so that, during use of the diaper 10, the liquid-absorbent structure 35 may be partially spaced apart from the interior surface of the crotch laminated sheet 34 and suspended therefrom.

Referring to Figs. 2 and 3, the crotch laminated sheet 34 is composed of interior and exterior crotch sheets 38, 39 at least one of which is formed of a liquid-impermeable fibrous nonwoven fabric sheet or a plastic film. The interior and exterior crotch sheets 38, 39 are bonded to each other with a hot melt adhesive (not shown) distributed to the interior surface of one of these two sheets 38, 39 and both lateral portions of these sheets 38, 39 are folded inwardly to define a pair of lateral elastic portions 40 extending in the longitudinal direction Y.

The respective lateral elastic regions 40 are provided with a plurality of strand or string first leg elastic elements 41 and second leg elastic elements 42 extending in the longitudinal direction Y and a plurality of strand or string second leg elastic elements 42 and thereby elasticized at least in the longitudinal direction Y. The first leg elastic elements 41 rectilinearly extend in the longitudinal direction Y along inner side edges 40a of the respective lateral elastic regions 40 and the second leg elastic elements 42 are curved inwardly in a midsection of the crotch region 15 and extend toward the front and rear waist regions 13, 14. The first and second leg elastic elements 41, 42 are attached under tension in the longitudinal direction Y between the both sheets 38, 39 with a hot melt adhesive (not shown) distributed to the interior surface of one of the interior and exterior crotch sheets 38, 39.

Respective middle segments 42a of the second leg elastic elements 42 are convexly curved toward the longitudinal axis P and consequently tensile stress of these elastic elements is locally enhanced. As a result, in vicinities of these middle segments facing the wearer's thighs, the lateral elastic regions 40 come in close contact with the wearer's body, thereby effectively preventing the body exudates from leaking sideways beyond the peripheral edges of the leg-openings.

The crotch panel 12 is attached to the respective exterior surfaces of the front and rear waist panels 16, 17 in a front bonding region 45 and a rear bonding region 46 formed of a hot melt adhesive distributed to the skin-facing surface of the front end portion 12A and the rear end portion 12B. By attaching the front and rear end portions 12A, 12B of the crotch panel 12 to the respective exterior surfaces of the front and rear waist panels 16, 17 in this manner, it is possible to ensure the body exudates containing space S larger than that obtained by attaching the front and rear end portions to the respective interior surfaces of the front and rear waist panels 16, 17 (See Fig. 6). In this regard, so long as the body exudates containing space S has a desired volume, only one of the front and rear end portions 12A and 12B may be attached to the exterior surface of the associated one of the front and rear waist panels 16, 17.

The front and rear bonding regions 45, 46 have a concave shape which opens toward the crotch region 15 and each of them has lateral portions 48 formed of a hot melt adhesive distributed to the skin-facing surface of the lateral elastic regions 40 and a midportion 49 extending in the transverse direction X between the lateral portions 48. The midportion 49 extends outboard of an existence region of the liquid-absorbent structure 35 as viewed in the longitudinal direction Y and a non-bonding region 50 to which no hot melt adhesive is distributed is defined between the lateral portions 48 and the midportion 49. While the lateral portions 48 of the front bonding region 45 are stepwise and the lateral portions 48 of the rear bonding region 46 are rectangular according to the present embodiment, these lateral portions 48 of the front and rear bonding regions 45, 46 are not limited to such shape and may have other various shapes such as stepwise, rectangular or curved shape.

The front and rear bonding regions 45, 46 respectively define the non-bonding regions 50 between the lateral portions 48 and the midportions 50 in this manner and, in consequence, front and rear pockets (spatial portions) 51, 52 are formed in these non-bonding regions 50 between the front and rear waist panels 16, 17 and the crotch panel 12. The front and rear pockets 51, 52 formed in this manner make it possible to enlarge a volume of the body exudates containing space S and, in addition, enable the crotch panel 12 to be suspended from the elastic waist panel 11 under its own mass of the body exudates, as will be described in detail.

Referring to Fig. 4, the liquid-absorbent structure 35 includes so-called absorbent material 60 formed only of superabsorbent polymer particles (sometimes referred to simply as absorbent polymer particles) which are water-soluble and have an absorption performance corresponding to ten times or higher of their own mass, an upper sheet 61 lying on the side of the skin-facing surface and formed of a water-permeable fibrous nonwoven fabric having a mass per unit area in a range of about 8 to about 15 g/m², preferably of about 10 g/m² and a lower sheet 62 lying on the side of the non-skin-facing surface and formed of a water-permeable or poorly-water-permeable SMS fibrous nonwoven fabric having a mass per unit area in a range of about 8 to about 15 g/m², preferably of about 11 g/m².

The liquid-absorbent structure 35 has, in addition, a plurality of substantially rectangular liquid-absorbent regions 63 spaced apart from each other in the longitudinal direction Y by a given dimension and a non-liquid-absorbent region 64 formed to surround the respective liquid-absorbent regions 63 and containing substantially none of the absorbent material 60. While the liquid-absorbent regions 63 are composed of eight compartments according to the present embodiment, the total area and the number of the compartments of the liquid-absorbent regions 63 may be appropriately changed depending on the absorption performance required for the liquid-absorbent structure 35. Furthermore, so long as the advantageous effects of the present invention as will be described later in detail can be ensured, the absorbent material 60 may include, in addition to the superabsorbent polymer particles, other known materials such as fluff wood pulp and/or thermoplastic fibers as a part of the absorbent materials.

In the liquid-absorbent regions 63, the superabsorbent polymer particles of a mass per unit area in a range of about 30 to about 300 g/m², preferably in a range of about 40 to about 280 g/m² are almost uniformly attached with a hot melt adhesive 66 distributed to the interior surface of the lower sheet 62. In the liquid-absorbent regions 63, preferably, the upper sheet 61 and the lower sheet 62 are partially attached to each other or not attached at all to each other.

In the non-liquid-absorbent region 64, more specifically, in spacing zones located between each pair of the liquid-absorbent regions 63 and a marginal zone located along an outer peripheral edges of the liquid-absorbent structure 35, the upper and lower sheets 61, 62 are bonded to each other with a hot melt adhesive 66.

In this manner, the liquid-absorbent structure 35 is formed from the absorbent material 60 composed of the superabsorbent polymer particles only and a sheet member used to wrap them to have a sheet-like configuration as a whole. Consequently, the liquid-absorbent structure 35 has a thickness smaller than the case in which the absorbent material is a mixture of superabsorbent polymer particles and fluff woodpulp, and assures a correspondingly high flexibility so as to readily conform to movements of the crotch panel. In addition, the upper and lower sheets 61, 62 stably attached to each other in the non-absorbent region 64 ensure a required peel strength and a flexibility higher than the case in which the interior surfaces of the upper and lower sheets 61, 62 are attached to each other over their entire areas. Furthermore, the superabsorbent polymer particles are evenly distributed and attached in the liquid-absorbent region 63 and, in consequence, the distribution of the superabsorbent polymer particles should not be biased due to movements and postures of the wearer.

An alternative embodiment is effective to assure the advantageous effects of the present invention. Specifically, the permissible maximum quantity of the superabsorbent polymer particles are distributed in the liquid-absorbent region 63 but partially not attached to the liquid-absorbent region 63 with the hot melt adhesive 66 and movable in the liquid-absorbent region 63. In this case, the liquid-absorbent region 63 will have a pouch-like configuration and the maximum quantity of the superabsorbent polymer particles containable therein will be a mass per unit area of about 400 g/m². In such alternative arrangement, the non-absorbent region 64 may function as a sealing region to seal the periphery of the liquid-absorbent region 63. However, there is a possibility that the superabsorbent polymer particles might partially migrate into the non-absorbent region 64 in the course of production process within a negligible range compared to the required mass per unit area of the superabsorbent polymer particles in the liquid-absorbent region 63.

A thickness of the crotch panel 12 in a midsect ion thereof including the liquid-absorbent structure 35 is specifically about 5.0 mm or less. A cantilever bending resistance thereof is in a range of about 15 to about 140 mm.

### <Method of thickness measurement>

The thickness of the crotch panel 12 may be measured with use of Thickness Tester (a diameter of a gauge head is in a range of about 10 to about 20 mm) (manufactured by PEACOCK CO. LTD.).

### <Measuring method for cantilever bending resistance>

According to Cantilever Method prescribed in JIS L1096, samples (a length dimension of 50 mm in the transverse direction X and a length dimension of 150 mm in the longitudinal direction Y) are cut out from the midsection of the crotch panels 12 of the respective diapers 10 and measurement is carried out on the skin-facing surface and the non-skin-facing surface of the respective samples. The number of times (n) of the measurement is 3 (three).

So long as the liquid-absorbent structure 35 has the required level of liquid-absorption performance and the required flexibility assured by the above-described thickness dimension, the liquid-absorbent structure 35 is not limited to the structure laid out into the liquid-absorbent region 63 and the non-liquid-absorbent region 64, but also may be the sheet-like structure formed with the liquid-absorbent region 63 in its entire area.

Referring to Fig. 5, a length dimension L2 (i.e., a length dimension of the outer periphery of the containing space S) between the inner side edge 40a of one of the lateral elastic regions 40 and the inner side edge 40a of the other of the lateral elastic region 40 is in a range of about 380 to about 450 mm. During use of the diaper 10, the lateral elastic regions 40 are spaced apart from the liquid-absorbent structure 35 and come in contact with inguinal regions of the wearer and the crotch panel 12 is spaced apart from the liquid-absorbent structure 35 and suspended from the elastic waist panel 11 in a bag-like configuration. When body exudates are discharged into the crotch panel 12 in this state of the diaper 10 put on the wearer' s body, the crotch panel 12 is spaced downwardly from the wearer's buttocks under its own mass and the body waist containing space S having a volume larger than that in the conventional disposable diapers is formed between the wearer's body and the liquid-absorbent structure 35. The diaper 10 has such body exudates containing space S having the volume larger than that in the conventional disposable diapers, whereby a relatively large volume of body exudates may be contained and, in addition, the crotch panel 12 is spaced apart from the wearer's buttocks and suspended like a hammock to prevent the wearer's buttocks from being soiled with body exudates.

Referring to Fig. 6, the liquid-absorbent structure 35 is in the sheet-like configuration having a relatively small thickness and relatively low stiffness since the absorbent material 60 therein is composed of the superabsorbent polymer particles only. Consequently, the liquid-absorbent structure 35 may readily conform to movements of the wearer and, during use of the diaper 10, the liquid-absorbent structure 35 is in a state of being suspended within the crotch panel 12. In this state, the liquid-absorbent structure 35 is capable of absorbing bodily exudates not only through the skin-facing surface but also the non-skin-facing surface at a high efficiency. In addition, the liquid-absorbent structure 35 has a relatively high flexibility and, in consequence, may readily conform to movements of the crotch laminated sheet 34. More specifically, the lateral elastic regions 40 are elongated to form leakage-barriers 68 along which the liquid-absorbent structure 35 is curved so that the crotch panel 12 as a whole may have a bag-like configuration more swollen than the state before excretion.

Fig. 7 illustrates how to conduct a measurement relating to an evaluation of sagging of the crotch region 15 of the diaper 10 after excretion. This evaluation was conducted by the following steps as described below.

First, the diaper 10 (of L-size) was put on an air doll 70 and the lowest position D1 of the crotch region 15 (from the waist-opening periphery 21a to the lowest portion) was recorded. Then 150 g of artificial urine was injected into the diaper 10 and the lowest position D2 of the crotch region 15 after three minutes had elapsed was recorded. Finally, the lowest position D1 recorded at the beginning was compared to the lowest position D2 of the crotch region 15 after three minutes had elapsed after injection of the artificial urine to measure a distance of downward movement T (mm) corresponding to the distance of downward movement after excretion. The air doll 70 was selected on the assumption of a physical type of the standard adult (regardless of gender), specifically, having a length dimension of about 73 mm around the waist, a length dimension of about 90 mm around the buttocks and a length dimension of about 49 mm around each of the thighs. An amount of the artificial urine injected into the diaper 10 is selected on the basis of an average amount of urine, about 150 g, discharged by the adult at each urination.

Based on the measurement result, the movement distance T from the lowest position D1 to the lowest position D2 of the crotch region 15 was about 50 mm. The similar measurement made on the conventional diaper arranged in similar manner to the diaper 10 indicated that this movement distance T is in a range of about 10 to about 30 mm. In view of this, in the diaper 10 according to the present invention, the crotch panel 12 sags down from the position before excretion and is spaced apart from the wearer's body after excretion more significantly than in the conventional diaper. In consequence, the body exudates having been absorbed by the liquid-absorbent structure 35 of the crotch panel 12 and/or retained in the containing space S should not adhere to the wearer' s body and not create a feeling of discomfort, and whereby rash or eruption due to body exudates adhering to the wearer's skin for a long period should not be caused.

The vicinities of the respective inner side edges 40a of the lateral elastic regions 40 come in close contact with the wearer's inguinal regions and thereupon the lateral elastic regions 40 are spaced apart from the lower surface of the crotch panel 12 to define the leakage-barriers 68. Consequently, the body exudates should not leak sideways beyond the leg-openings' peripheries.

Such advantageous effects described above can be ensured because of the following features: the liquid-absorbent structure 35 has a sufficiently small thickness and a sufficiently low stiffness in order to readily conform to movements of the crotch laminated sheet 34; and the crotch panel 12 has its configuration like a swollen bag before an excretion occurs and, upon the excretion, sags down under its own mass of the body exudates. The effects can be attributed also to the dimensional factor such that the length dimension L2 from the inner side edge 40a of one of the lateral elastic regions 40 to the inner side edge 40a of the other lateral elastic region 40 defining an outer peripheral dimension of the containing space S is relatively large with respect to the width dimension (size) of the diaper 10. In view of this, so long as the diaper has such arrangement, the advantageous effect of the present invention can be ensured also in the case that the front waist regions 13, the rear waist region 14 and the crotch region 15 are not separately formedbut are formed froman integrated member.

The size of the containing space S relative to the size of the diaper 10 as a whole may be represented by a dimensional ratio (L2/L1) between the length dimension of the inner periphery of the waist-opening 21 as a reference index for the diaper size or the length dimension L1 of the waist-opening periphery 21a of the front and rear waist regions 13, 14 in the transverse direction X corresponding to 1/2 of the above-mentioned length dimension and the length dimension L2 measured from the inner side edge 40a of one of the lateral elastic regions 40 to the inner side edge 40a of the other lateral elastic region 40.

Specifically, if the dimensional ratio (L2/L1) is relatively small, it is meant that the containing space S within the crotch panel 12 is relatively small with respect to the dimension (the size) of the diaper 10 and, in consequence, the crotch panel 12 is unlikely to have a remarkably swollen shape. For this reason, even if the crotch panel 12 sags down under its own mass of the body exudates, the movement distance T thereof is too small to space the crotch panel 12 sufficiently from the wearer's body.

If the dimensional ratio (L2/L1) is large, it is meant that the containing space S having a sufficient absorption capacity with respect to the dimension (the size) of the diaper 10 and the crotch panel 12 has a largely swollen configuration already before the excretion. Consequently, upon excretion, the crotch panel 12 sags down by a relatively significant distance under its own mass of the body exudates and this movement distance T is sufficiently large to space the crotch panel 12 from the wearer's body, thereby preventing the body exudates from adhering to the wearer's body.

The length dimensional ratios (L2/L1) of the adult diapers 10 of respective sizes S to LL according to the present invention are listed in TABLE 1. The length dimension L2 measured from the inner side edge 40a of one of the lateral elastic regions 40 to the inner side edge 40a of the other lateral elastic region 40 according to the present invention is uniformly about 410 mm in any and every size. For the conventional adult diapers (S-size to LL-size), more specifically, those having the width dimension of the front waist region corresponding to L1 in the embodiment of the present invention in a range of about 400 to about 900 mm and the width dimension between the inner side edges of the leg-opening periphery corresponding to L2 in the embodiment of the present invention in a range of about 180 to about 250 mm, the similar measurement was made. According to the result of measurement, the dimensional ratio was about 0.37 on an average. In other words, the width dimension corresponding to L2 was approximately 0.37 times of the width dimension corresponding to L1.

| Sizes of adult diaper | Width dimension L1 (mm) of waist-opening periphery (only in front waist region) | Length dimension L2 (mm) between inner side edges of lateral elastic regions | Ratio of width dimensions (L2/L1) |
|---|---|---|---|
| S-size | 480 | 410 | 0.85 |
| M-size | 530 | 410 | 0.77 |
| L-size | 630 | 410 | 0.65 |
| LL-size | 730 | 410 | 0.56 |

Referring to TABLE 1, the width dimension ratios L2/L1 of the adult diapers 10 of the respective sizes are in a range of about 0.5 to about 1.0 and, that is, the length dimensions (L2) between the inner side edges 40a of the lateral elastic regions 40 are in a range of about 0.5 to about 1.0 times of the respective length dimensions L1. In this manner, compared to the conventional diaper, the diaper 10 has the length dimension L2 relatively larger with respect to the entire length of the diaper 10 and, in consequence, the crotch panel 12 has the shape sufficiently swollen already prior to occurrence of the excretion to define the containing space S of large containing capacity. With the arrangement as described just above, the distance of movement T between the bottom positions D1, D2 of the crotch region 15 is sufficiently large to space the crotch panel 12 downwardly from the wearer's body, thereby preventing an adherence of the body exudates to the wearer's body.

The constituent elements of the disposable diaper 10 are not limited to those described in the specification but other various types of material widely used in the relevant technical field may be used without limitation unless otherwise stated. The terms "first" and "second" used in the description and Claims of the present invention are used merely to distinguish similar elements, similar positions and the like.

### {Reference Signs List}

10 disposable wearing article (disposable diaper)
11 elastic waist panel
12 crotch panel
13 front waist region
14 rear waist region
15 crotch region
34 crotch laminated sheet (liquid-impermeable sheet)
35 liquid-absorbent structure
40 lateral elastic regions
40a inner side edges of lateral elastic regions
41 first leg elastic elements
42 second leg elastic elements
49 adhesive distributed regions (front and rear bonding regions)
50 no adhesive distributed regions (front and rear non-bonding regions)
51, 52 spatial portions (front and rear pockets)
X transverse direction
Y longitudinal direction

## Claims

1. A disposable wearing article having a longitudinal direction and a transverse direction being orthogonal to the longitudinal direction, including:
a skin-facing surface;
a non-skin-facing surface;
a front waist region (13);
a rear waist region (14); and
a crotch region (15) lying between the front and rear waist regions (13, 14),
wherein:
the front and rear waist regions (13, 14) are elastically contractible at least in the transverse direction;
the crotch region (15) is provided in lateral portions with a pair of lateral elastic regions (40) adapted to be elastically contractible in the longitudinal direction, and a liquid-absorbent structure (35) is located between the pair of lateral elastic regions (40); **characterized in that**
the pair of lateral elastic regions (40) are respectively provided with a plurality of string or strand leg elastic elements including first leg elastic elements (41) rectilinearly extending in the longitudinal direction along the respective inner side edges of the lateral elastic regions (40) and second leg elastic elements (42) arranged outboard of the first leg elastic elements (411) as viewed in the transverse direction and concavely curved inwardly in vicinities of the central portion of the crotch region (15),
wherein the front and rear waist regions (13, 14) are formed of an annular elastic waist panel (11) and the crotch region (15) is formed of a crotch panel (12) attached to the elastic waist panel (11),
wherein at least one of front and rear end portions of the crotch panel (12) is attached to an exterior surface of the elastic waist panel (11) by means of a bonding region having a concave shape which opens toward the crotch region (15) wherein the bonding region has lateral portions (48) formed of a hot melt adhesive distributed to the skin-facing surface of the lateral elastic regions (40) and a midportion (49) extending in the transverse direction (X) between the lateral portions (48), the midportion (49) extends outboard of an existence region of the liquid-absorbent structure (35) as viewed in the longitudinal direction (Y) and a non-bonding region (50) to which no hot melt adhesive is distributed is defined between the lateral portions (48) and the midportion (49) and, in the non-bonding region, a spatial portion is defined by the elastic waist panel (11) and the one end of the crotch panel (12),
- a cantilever bending resistance in a substantially central portion of the crotch region (15) including the liquid-absorbent structure (35) is in a range of about 15 to about 140 mm, measured according to Cantilever Method prescribed in JIS L1096; and
- a length dimension (L2) measured from an inner side edge of one of the lateral elastic regions (40) to an inner side edge of another lateral elastic region is in a range of about 0.5 to about 1.0 times of a length dimension in the transverse direction of a waist-opening periphery (L1) in one of the front and rear waist regions (13, 14) measured under tension in the transverse direction.

2. The wearing article according to claim 1, wherein the liquid-absorbent structure (35) is formed of superabsorbent polymer particles wrapped with a liquid-permeable sheet in a sheet-like configuration and a thickness dimension of the crotch region (15) in the substantially central portion thereof including the liquid-absorbent structure is (35) about 5.0 mm or less.

## Patentansprüche

1. Wegwerfbare Trageartikel mit einer Längsrichtung und einer zu der Längsrichtung orthogonalen Querrichtung, umfassend:
eine Haut zugewandte Fläche;
eine Haut abgewandte Fläche;
einen vorderen Taillenbereich (13);
einen hinteren Taillenbereich (14); und
einen Schrittbereich (15), der zwischen den vorderen und hinteren Taillenbereichen (13, 14) liegt, wobei:
der vordere und hintere Taillenbereich (13, 14) zumindest in die Querrichtung elastisch zusammenziehbar sind;
der Schrittbereich (15) in seitlichen Abschnitten mit einem Paar von seitlichen elastischen Bereichen (40) versehen ist, die angepasst sind in die Längsrichtung elastisch zusammenziehbar zu sein,
und wobei eine flüssigkeitsabsorbierende Struktur (35) zwischen dem Paar von seitlichen elastischen Bereichen (40) angeordnet ist; **dadurch gekennzeichnet, dass**
das Paar von seitlichen elastischen Bereichen (40) jeweils mit einer Vielzahl von Schnur- oder Strangbeinelastikelementen versehen sind, die erste Beinelastikelemente (41), die sich geradlinig in die Längsrichtung entlang der jeweiligen Innenseitenkanten der seitlichen elastischen Bereiche (40) erstrecken, und zweite Beinelastikelemente (42) umfassen, die aus der Querrichtung gesehen außerhalb der ersten Beinelastikelemente (411) und im Bereich des zentralen Abschnitts des Schrittbereichs (15) konkav nach innen gekrümmt angeordnet sind,
wobei der vordere und hintere Taillenbereich (13, 14) aus einem ringförmigen elastischen Taillenpaneel (11) gebildet sind und der Schrittbereich (15) aus einem Schrittpaneel (12) gebildet ist, das an dem elastischen Taillenpaneel (11) befestigt ist,
wobei zumindest einer der vorderen und hinteren Endabschnitten des Schrittpaneels (12) an eine äußere Oberfläche des elastischen Taillenpaneels (11) mittels eines Klebebereichs befestigt ist, der eine konkave Form hat, die sich in Richtung des Schrittbereichs (15) öffnet, wobei der Klebebereich seitliche Abschnitte (48) hat, die aus einem Heißschmelzkleber gebildet sind, der auf der Haut zugewandten Fläche der seitlichen elastischen Bereiche (40) verteilt ist, und sich ein Mittelabschnitt (49) in die Querrichtung (X) zwischen den seitlichen Abschnitten (48) erstreckt, sich der Mittelabschnitt (49) aus der Längsrichtung (Y) gesehen außerhalb eines Existenzbereiches der flüssigkeitsabsorbierenden Struktur (35) erstreckt und ein Nichtklebebereich (50), auf den kein Heißschmelzkleber verteilt ist, zwischen den seitlichen Abschnitten (48) und dem Mittelabschnitt (49) bestimmt ist und wobei in dem Nichtklebebereich ein räumlicher Abschnitt durch das elastische Taillenpaneel (11) und dem einen Ende des Schrittpaneels (12) bestimmt ist,
- ein Auslegerbiegewiderstand, gemessen nach der in JIS L1096 vorgeschriebenen Auslegermethode, in einem im Wesentlichen zentralen Abschnitt des Schrittbereichs (15) umfassend die flüssigkeitsabsorbierende Struktur (35) in einem Intervall von ungefähr 15 bis ungefähr 140 mm ist; und
- eine Längenabmessung (L2), die von einer Innenseitenkante einer der seitlichen elastischen Bereiche (40) zu einer Innenseitenkante eines anderen seitlichen elastischen Bereichs gemessen, in einem Intervall von ungefähr 0,5 bis ungefähr 1,0 mal eine Längendimension in die Querrichtung eines Taillenöffnungsumfangs (L1) ist, die unter Spannung in die Querrichtung in einer der vorderen und hinteren Taillenbereichen (13, 14) gemessen ist.

2. Trageartikel nach Anspruch 1, wobei die flüssigkeitsabsorbierende Struktur (35) aus superabsorbierenden Polymerpartikeln gebildet ist, die in eine flüssigkeitsdurchlässige Lage in einer Lagenförmigen Anordnung gewickelt sind und wobei eine Dickenabmessung des Schrittbereichs (15) in seinem die flüssigkeitsabsorbierende Struktur (35) umfassenden, im Wesentlichen zentralen Abschnitt ungefähr 5,0 mm oder weniger ist.

## Revendications

1. Un article d'usure jetable ayant une direction longitudinale et une direction transversale étant orthogonale à la direction longitudinale, comprenant:
une surface exposée à la peau;
une surface non orientée vers la peau;
une région de la taille avant (13);
une région de la taille arrière (14); et
une région d'entrejambe (15) située entre les régions de ceinture avant et arrière (13, 14)
dans lequel:
les régions de la taille avant et arrière (13, 14) sont contractibles élastiquement au moins dans la direction transversale;
la région d'entrejambe (15) est prévue en portions latérales avec une paire de régions élastiques latérales (40) adaptées pour être contraintes élastiquement dans la direction longitudinale et une structure absorbant les liquides (35) est située entre les deux régions élastiques latérales (40) **caractérisé en ce que** la paire de régions élastiques latérales (40) sont pourvues respectivement d'une pluralité d'éléments élastiques de jambe de chaîne ou de brin comprenant des éléments élastiques (41) de premier segment s'étendant de manière rectiligne dans la direction longitudinale le long des bords latéraux intérieurs respectifs des régions élastiques latérales (40) et des éléments élastiques de deuxième étape (42) disposés à l'extérieur des premiers éléments élastiques (411) de la jambe, comme on les voit dans la direction transversale et incurvées de façon concave vers l'intérieur dans les environs de la partie centrale de la région d'entrejambe (15)
dans lequel les régions de ceinture avant et arrière (13, 14) sont formées d'un panneau de taille élastique annulaire (11) et la région d'entrejambe (15) est formée d'un panneau d'entrejambe (12) fixé au panneau de taille élastique (11)
dans lequel au moins l'une des parties d'extrémité avant et arrière du panneau d'entrejambe (12) est fixée à une surface extérieure du panneau de taille élastique (11) au moyen d'une région de liaison ayant une forme concave qui s'ouvre vers la région d'entrejambe (15) dans lequel la région de liaison présente des parties latérales (48) formées d'un adhésif thermofusible distribué sur la surface faisant face à la peau des régions élastiques latérales (40) et une partie intermédiaire (49) s'étendant dans la direction transversale (X) entre les parties latérales (48), la partie intermédiaire (49) s'étend à l'extérieur d'une région d'existence de la structure absorbant les liquides (35) telle que vue dans la direction longitudinale (Y) et une région sans liaison (50) à laquelle aucun adhésif thermofusible n'est distribué est définie entre les parties latérales (48) et la partie intermédiaire (49) et, dans la région sans liaison, une partie spatiale est définie par le panneau de taille élastique (11) et l'extrémité du panneau d'entrejambe (12),
- une résistance à la flexion en porte-à-faux dans une partie sensiblement centrale de la région d'entrejambes (15), y compris la structure absorbant les liquides (35), se situe dans une plage d'environ 15 à environ 140 mm, mesurée selon la méthode Cantilever prescrite dans JIS L1096; et
- une dimension de longueur (L2) mesurée à partir d'un bord latéral intérieur de l'une des régions élastiques latérales (40) jusqu'à un bord latéral interne d'une autre région élastique latérale est dans une plage d'environ 0,5 à environ 1,0 fois d'une dimension de longueur dans le direction transversale d'une périphérie d'ouverture de la taille (L1) dans l'une des régions de la taille avant et arrière (13, 14) mesurée sous tension dans la direction transversale.

2. L'article d'usure selon la revendication 1, dans lequel la structure absorbante de liquide (35) est formée de particules de polymère superabsorbant enveloppées avec une feuille perméable aux liquides dans une configuration en forme de feuille et une dimension d'épaisseur de la région d'entrejambe (15) dans la partie sensiblement centrale de celle-ci comprenant la structure absorbant les liquides (35) est d'environ 5,0 mm ou moins.
